# EUROPEAN PATENT SPECIFICATION

(11) **EP 3 634 456 B1**
(45) Date of publication and mention of the grant of the patent: **22.11.2023**
(21) Application number: 17748916.8
(22) Date of filing: 19.06.2017
(51) Int. Cl.: A61K 36/88, A61K 36/9066, A61K 38/48, A61K 45/06, A61K 31/05, A61K 31/07, A61K 31/122, A61K 31/125, A61K 31/4525, A61K 36/02, A61K 36/28, A61K 36/324, A61K 36/67, A61K 36/704, A61P 9/10, A61P 27/02

(54) **COMPOSITION FOR USE IN TREATING RETINAL DISEASES**
ZUSAMMENSETZUNG ZUR VERWENDUNG BEI DER BEHANDLUNG VON NETZHAUTERKRANKUNGEN
COMPOSITION UTILISABLE DANS LE TRAITEMENT DE RÉTINOPATHIES

(30) Priority: 09.06.2017 IT 201700063303
(43) Date of publication of application: 15.04.2020
(73) Proprietor: SIFI S.p.A., 95025 Aci Sant'Antonio (CT) (IT)
(72) Inventor: SANTOCONO, Marcello, 95020 Pisano-Zaffernana Etnea, Catania (IT); SANTONOCITO, Manuela, Aci Sant`Antonio 95025 Catania (IT); ZAPPULLA, Cristina, 95025 Aci Sant'Antonio (Catania) (IT); LA ROSA, Luca Rosario, 95025 Aci Sant'Antonio (Catania) (IT); GIULIANO, Francesco, 95025 Aci Sant'Antonio (Catania) (IT); MAZZONE, Maria Grazia, 95025 Aci Sant'Antonio (Catania) (IT)
(74) Representative: Cattaneo, Elisabetta
(86) International application number: PCT/IB2017/053617
(87) International publication number: WO 2018/224869

(56) References cited:
- GB-A- 2 483 121
- US-A- 5 536 506
- US-A1- 2013 231 297
- SCHMIDL D ET AL: "Nutritional supplements in age-related macular degeneration", ACTA OPHTHALMOLOGICA: THE OPHTHALMOLOGICAL JOURNAL OF THE NORDIC COUNT, WILEY-BLACKWELL MUNKSGAARD, DENMARK, vol. 93, no. 2, 1 March 2015 (2015-03-01), pages 105-121, XP009189404, ISSN: 1755-375X, DOI: 10.1111/AOS.12650
- MCCUSKER MEAGEN M ET AL: "An eye on nutrition: The role of vitamins, essential fatty acids, and antioxidants in age-related macular degeneration, dry eye syndrome, and cataract", CLINICS IN DERMATOLOGY, J.B. LIPPINCOTT, PHILADELPHIA, PA, US, vol. 34, no. 2, 22 November 2015 (2015-11-22), pages 276-285, XP029438711, ISSN: 0738-081X, DOI: 10.1016/J.CLINDERMATOL.2015.11.009
- LULLI MATTEO ET AL: "Acetyl-11-keto-[beta]-boswellic acid reduces retinal angiogenesis in a mouse model of oxygen-induced retinop", EXPERIMENTAL EYE RESEARCH, ACADEMIC PRESS LTD, LONDON, vol. 135, 23 April 2015 (2015-04-23), pages 67-80, XP029243617, ISSN: 0014-4835, DOI: 10.1016/J.EXER.2015.04.011
- Chunyan Xue ET AL: "Management of Ocular Diseases Using Lutein and Zeaxanthin: What Have We Learned from Experimental Animal Studies?", Journal of Ophthalmology, vol. 2015, 1 January 2015 (2015-01-01), pages 1-11, XP055761905, US ISSN: 2090-004X, DOI: 10.1155/2015/523027

## Description

A composition is described herein, which comprises: astaxanthin, piperine, boswellic acids, coenzyme Q10, curcuminoids, lutein, zeaxanthin, saffron, copper and, optionally, resveratrol, bromelain, zinc, salicin and further antioxidants from natural sources. The present invention further relates to said composition for use in treating retinal diseases which are ischemic and/or degenerative in nature.

### Background art

The retina is the innermost and most differentiated of the 3 tunics which form the wall of the eyeball, extending from the optic nerve to the pupil edge of the iris. It has the function of receiving the light stimuli and transform them into nerve signals which are transmitted up to the brain structures.

From the histological point of view, the retina is made up of two layers:
1. the retinal pigment epithelium or outer leaflet
2. the nervous layer or inner leaflet or retina properly said.

The retina consists of several cell layers and is divided into two areas: a central area, called macula, rich in cones, and a medium and peripheral area, where rod cells prevail. The vasculature of the eye is ensured by two structurally and functionally separate systems, the retinal system and the ciliary system. Injuries and illnesses which impair the normal structure or function of these pathways are among the leading causes of vision impairment and blindness. For example, diabetic retinopathy is the most common disease affecting the retinal vasculature, and is the leading cause of sight loss among the working age population.

Astaxanthin is a carotenoid which can be extracted from various sources including the *Haematococcus sp.,* a green alga with high antioxidant power.

Piperine is an alkaloid present in *Piper nigrum.*

Boswellic acids are obtained from *Boswellia serrata.*

Bromelain is present in the extracts of *Ananas comosus* and includes two proteolytic enzymes.

Coenzyme Q10 is a powerful antioxidant.

Curcuminoids are present in the extract of *Curcuma longa.*

Lutein belongs to the group of xanthophylls, can be extracted from various sources including the flowers of *Tagetes erecta.*

Resveratrol is a polyphenol found in the skin of the grape and is successfully extracted from various sources, including the roots of *Polygonum cuspidatum.*

Salicin, found in plants of the *Salicacae* family, is a β-glucoside with anti-Cox activity.

Saffron is extracted from the stigmas of *Crocus sativus.*

Zeaxanthin is a carotenoid of the xanthophylls class which can be extracted from various sources, including the flowers of *Tagetes erecta.*

GB2483121 dicloses compositions that can be added to dietary formulations for use in treating age-related macular degeneration which include saffron and resveratrol as main ingredients combined with zinc, copper, lutein, coenzyme Q10 and zeaxanthin besides other antioxidants.These compositions do not include piperine, astaxanthin, boswellic acids or curcuminoids.

There is an unmet need for complementary treatment methods and compositions directed to retinal diseases. The present invention meets this need.

### Description of the invention

A composition comprising natural extracts, carotenoids and minerals is described. Such a composition proved surprisingly capable of protecting the retina by promoting functional recovery in post-ischemic stress conditions.

### Description of the drawings

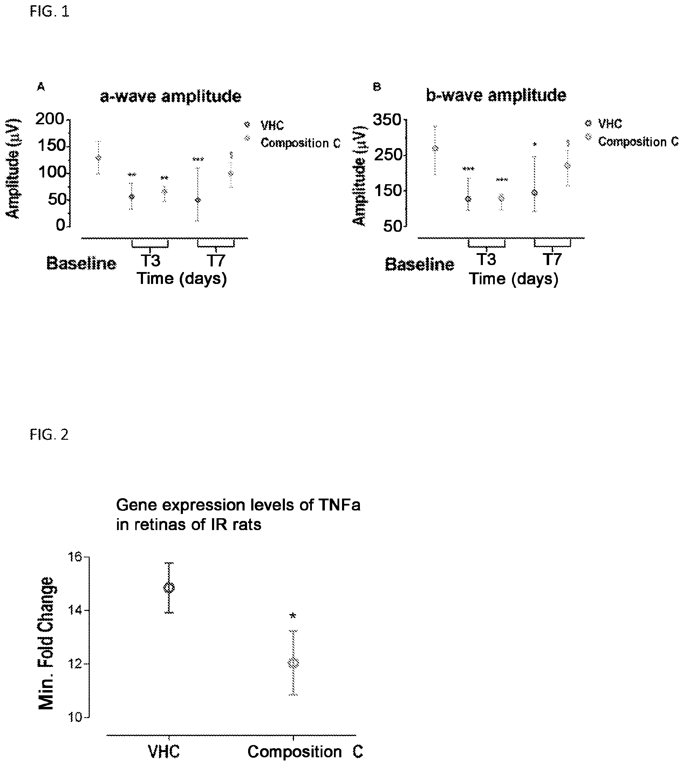

**Figure 1****:** Comparison of ERG recordings of wave a (A) and b (B) with respect to the Baseline at T3 and T7 in rats pretreated orally once daily through tube feeding with composition C according to the present invention or VHC (vehicle) for 5 days, 1h before induction of IR and treated for the next 7 days. The data represent the average and the range of at least 4 animals per treatment group. *p≤0.05; **p≤0.01; ***p≤0001; treatment vs. Baseline. §p≤0.05 vs. VHC. One-way ANOVA followed by the post hoc test of Holm-Amshid.
**Figure 2****:** Gene expression of TNFa in retinas of rats pretreated orally once daily through tube feeding with composition C according to the present invention or VHC (vehicle) for 5 days and 1h before induction of IR and sacrificed 6h after the ischemic event. The data represent the mean ± SEM of Fold Changes with respect to the calibrator (CTRL NAÏVE). *p≤0.05 vs. VHC. Unpaired t-test.

### Detailed description of the invention

In one embodiment, the composition according to the present invention comprises: astaxanthin, piperine, boswellic acids, coenzyme Q10, curcuminoids, lutein, copper, saffron, zeaxanthin. In a further embodiment, the composition comprises also: resveratrol, bromelain, zinc, salicin.

In a further embodiment, said composition comprises further antioxidants from natural sources.

More preferably, said further natural antioxidants are polymeric anthocyanins derived from blackcurrant.

In one embodiment, said composition comprises: 0.1 to 200 mg of concentrated extract from *Haematococcus sp.* containing no less than 2.0% astaxanthin, 0.01 to 15 mg dry extract, titered at 95% piperine obtained from fruits of *Piper nigrum,* 10 to 2000 mg of extract from gum resin of *Boswellia serrata* titered at 70% total boswellic acids and 20% beta boswellic acids, 0 to 500 mg of extract from stem and fruit of *Ananas comosus* containing bromelain equivalent to gelatin digesting units (GDU) of between 1 and 1000, 0.5 to 100 mg of coenzyme Q10, 1 to 500 mg dry extract from the root of *Curcuma longa* titered at 95% total curcuminoids, 0.1 to 250 mg extract from flowers of *Tagetes erecta* titered at 20% lutein, 0 to 500 mg dry extract from *Ribes nigrum* with an oxygen radical absorption capacity (ORAC) of no less than 2 mmol trolox equivalents per gram of extract, 0.25 to 0.75 mg of copper (from microencapsulated copper sulfate), 0 to 150 mg of resveratrol purified from roots of *Polygonum cuspidatum,* 0 to 200 mg dry extract of bark of *Salix alba* titered at 25% salicin, 1 to 100 mg of dry extract from stigmas of *Crocus sativus* titered at 2% safranal, 0.1 to 30 mg of extract from flowers of *Tagetes erecta* titered at 5% zeaxanthin, 0 to 10 mg of zinc (from zinc oxide).

In a further embodiment, said composition comprises: 0.5 to 150 mg of concentrated extract from *Haematococcus sp.* containing no less than 2.0% astaxanthin, 0.1 to 10 mg dry extract, titered at 95% piperine obtained from fruits of *Piper nigrum,* 20 to 1500 mg of extract from gum resin of *Boswellia serrata* titered at 70% total boswellic acids and 20% beta boswellic acids, 2 to 400 mg of extract from stem and fruit of *Ananas comosus* containing bromelain equivalent to gelatin digesting units (GDU) of between 1 and 1000, 1 to 80 mg of coenzyme Q10, 5 to 400 mg dry extract from the root of *Curcuma longa* titered at 95% total curcuminoids, 0.1 to 200 mg extract from flowers of *Tagetes erecta* titered at 20% lutein, 0 to 250 mg dry extract from *Ribes nigrum* with an oxygen radical absorption capacity (ORAC) of no less than 2 mmol trolox equivalents per gram of extract, 0.25 to 0.75 mg of copper (from microencapsulated copper sulfate), 1.5 to 100 mg of resveratrol purified from roots of *Polygonum cuspidatum,* 10 to 150 mg dry extract of bark of *Salix alba* titered at 25% salicin, 2 to 50 mg of dry extract from stigmas of *Crocus sativus* titered at 2% safranal, 0.2 to 20 mg of extract from flowers of *Tagetes erecta* titered at 5% zeaxanthin, 2.5 to 7.5 mg of zinc (from zinc oxide).

In a further embodiment, said composition comprises: 1 to 100 mg of concentrated extract from *Haematococcus sp.* containing no less than 2.0% astaxanthin, 0.2 to 8 mg dry extract, titered at 95% piperine obtained from fruits of *Piper nigrum,* 25 to 1000 mg of extract from gum resin of *Boswellia serrata* titered at 70% total boswellic acids and 20% beta boswellic acids, 5 to 300 mg of extract from stem and fruit of *Ananas comosus* containing bromelain equivalent to gelatin digesting units (GDU) of between 8 and 480, 3 to 75 mg of coenzyme Q10, 10 to 250 mg dry extract from the root of *Curcuma longa* titered at 95% total curcuminoids, 1 to 150 mg extract from flowers of *Tagetes erecta* titered at 20% lutein, 0 to 125 mg dry extract from *Ribes nigrum* with an oxygen radical absorption capacity (ORAC) of no less than 2 mmol trolox equivalents per gram of extract, 0.3 to 0.75 mg of copper (from microencapsulated copper sulfate), 3 to 75 mg of resveratrol purified from roots of *Polygonum cuspidatum,* 30 to 100 mg dry extract of bark of *Salix alba* titered at 25% salicin, 4 to 40 mg of dry extract from stigmas of *Crocus sativus* titered at 2% safranal, 0.5 to 18 mg of extract from flowers of *Tagetes erecta* titered at 5% zeaxanthin, 3 to 7.5 mg of zinc (from zinc oxide).

In a further embodiment, said composition comprises: 1 to 80 mg of concentrated extract from *Haematococcus sp.* containing no less than 2.0% astaxanthin, 0.5 to 5 mg dry extract, titered at 95% piperine obtained from fruits of *Piper nigrum,* 40 to 800 mg of extract from gum resin of *Boswellia serrata* titered at 70% total boswellic acids and 20% beta boswellic acids, 10 to 250 mg of extract from stem and fruit of *Ananas comosus* containing bromelain equivalent to gelatin digesting units (GDU) of between 8 and 480, 3.5 to 50 mg of coenzyme Q10, 15 to 200 mg dry extract from the root of *Curcuma longa* titered at 95% total curcuminoids, 3 to 100 mg extract from flowers of *Tagetes erecta* titered at 20% lutein, 16 to 100 mg dry extract from *Ribes nigrum* with an oxygen radical absorption capacity (ORAC) of no less than 2 mmol trolox equivalents per gram of extract, 0.35 to 0.65 mg of copper (from microencapsulated copper sulfate), 6 to 50 mg of resveratrol purified from roots of *Polygonum cuspidatum,* 35 to 95 mg dry extract of bark of *Salix alba* titered at 25% salicin, 5 to 25 mg of dry extract from stigmas of *Crocus sativus* titered at 2% safranal, 1 to 17 mg of extract from flowers of *Tagetes erecta* titered at 5% zeaxanthin, 3 to 7 mg of zinc (from zinc oxide).

In a further embodiment, said composition comprises: 5 to 50 mg of concentrated extract from *Haematococcus sp.* containing no less than 2.0% astaxanthin, 0.75 to 4 mg dry extract, titered at 95% piperine obtained from fruits of *Piper nigrum,* 50 to 600 mg of extract from gum resin of *Boswellia serrata* titered at 70% total boswellic acids and 20% beta boswellic acids, 20 to 200 mg of extract from stem and fruit of *Ananas comosus* containing bromelain equivalent to gelatin digesting units (GDU) of between 15 and 370, 3 to 25 mg of coenzyme Q10, 30 to 100 mg dry extract from the root of *Curcuma longa* titered at 95% total curcuminoids, 10 to 50 mg extract from flowers of *Tagetes erecta* titered at 20% lutein, 20 to 60 mg dry extract from *Ribes nigrum* with an oxygen radical absorption capacity (ORAC) of no less than 2 mmol trolox equivalents per gram of extract, 0.35 to 0.65 mg of copper (from microencapsulated copper sulfate), 8 to 25 mg of resveratrol purified from roots of *Polygonum cuspidatum,* 35 to 95 mg dry extract of bark of *Salix alba* titered at 25% salicin, 6 to 20 mg of dry extract from stigmas of *Crocus sativus* titered at 2% safranal, 2 to 15 mg of extract from flowers of *Tagetes erecta* titered at 5% zeaxanthin, 4 to 7 mg of zinc (from zinc oxide).

In a further embodiment, said composition comprises: 10 to 30 mg of concentrated extract from *Haematococcus sp.* containing no less than 2.0% astaxanthin, 1 to 3 mg dry extract, titered at 95% piperine obtained from fruits of *Piper nigrum,* 60 to 200 mg of extract from gum resin of *Boswellia serrata* titered at 70% total boswellic acids and 20% beta boswellic acids, 30 to 100 mg of extract from stem and fruit of *Ananas comosus* containing bromelain equivalent to gelatin digesting units (GDU) of between 50 and 200, 4 to 12 mg of coenzyme Q10, 30 to 70 mg dry extract from the root of *Curcuma longa* titered at 95% total curcuminoids, 15 to 35 mg extract from flowers of *Tagetes erecta* titered at 20% lutein, 20 to 40 mg dry extract from *Ribes nigrum* with an oxygen radical absorption capacity (ORAC) of no less than 2 mmol trolox equivalents per gram of extract, 0.4 to 0.6 mg of copper (from microencapsulated copper sulfate), 10 to 20 mg of resveratrol purified from roots of Polygonum cuspidatum, 40 to 90 mg dry extract of bark of Salix alba titered at 25% salicin, 7 to 15 mg of dry extract from stigmas of *Crocus sativus* titered at 2% safranal, 5 to 15 mg of extract from flowers of *Tagetes erecta* titered at 5% zeaxanthin, 5 to 7 mg of zinc (from zinc oxide).

The composition according to the present invention has proved surprisingly effective in inhibiting the expression of TNFa and promote the retinal functional recovery in an experimental model *in vivo.*

This activity is specifically obtained with the composition of the present invention. The different components, when tested individually, are not able to give the effect obtained with the composition, demonstrating a clear synergistic effect between the tested components.

The composition according to the present invention comprises natural extracts and provides a valuable complement in the treatment of retinal diseases.

The present invention further relates to an oral formulation combining a composition according to the present invention, as described in claims 1 to 6, with pharmaceutically acceptable excipients known in the art. These excipients allow the compositions of the invention to be formulated as powders, tablets, pills, capsules, liquids, gels, syrups, semiliquid mixtures, suspensions and the like for oral ingestion by an individual. The pharmaceutical preparations for oral use can be obtained as a solid excipient, optionally by grinding a resulting mixture, and processing the mixture of granules, after adding suitable adjuvants, if desired, to obtain tablets or pill cores. In particular, suitable excipients are fillers such as sugars, including lactose or sucrose; cellulose preparations such as maize starch, wheat starch, rice starch, potato starch, gelatin, tragacanth, methylcellulose, hydroxypropyl methylcellulose, sodium carboxymethylcellulose, and/or polyvinylpyrrolidone (PVP); and various flavoring agents known in the art, such as cocoa. If desired, disintegrating agents may be added, such as cross-linked polyvinylpyrrolidone, agar, or alginic acid or a salt thereof, such as sodium alginate. Said formulations may be administered in the form of a chewable tablet or of a tablet which disintegrates in the mouth, liquid, powder, powder contained in a sachet, pills, soft gelatin capsule, or hard gelatin capsule. In some embodiments, the compositions of the invention are provided as pharmaceutical compositions as in the form of liquid compositions. Suitable liquid excipients are known in the art; see, for example, Remington's Pharmaceutical Sciences.

A method is also described which is an adjuvant in the treatment of retinal diseases including the administration, to a person who is in need thereof, of the composition according to the present invention, as described in claims 1 to 6. Said composition is administered in a dosage of between 50 mg to 5 g/day, preferably between 100 mg and 2.5 g/day, or between 200 mg and 1.5 g/day, even more preferably between 300 mg and 800 mg/day.

### Examples

### Example 1: Compositions.

Compositions according to the present invention were prepared as follows.

### Composition A

20 mg of concentrated extract from *Haematococcus sp.* containing no less than 2.0% astaxanthin
2.5 mg dry extract, titered at 95% piperine obtained from fruits of *Piper nigrum*
100 mg of extract from gum resin of *Boswellia serrata* titered at
70% total boswellic acids and 20% beta boswellic acids
5 mg of coenzyme Q10
50 mg dry extract from the root of *Curcuma longa* titered at 95% total curcuminoids
25 mg extract from flowers of *Tagetes erecta* titered at 20% lutein
0.5 mg of copper (from microencapsulated copper sulfate)
10 mg dry extract from stigmas of *Crocus sativus* titered at 2% safranal
10 mg extract from flowers of *Tagetes erecta* titered at 5% zeaxanthin

### Composition B

20 mg of concentrated extract from *Haematococcus sp.* containing no less than 2.0% astaxanthin
2.5 mg dry extract, titered at 95% piperine obtained from fruits of *Piper nigrum*
100 mg of extract from gum resin of *Boswellia serrata* titered at 70% total boswellic acids and 20% beta boswellic acids
81 mg extract from stem and fruit of *Ananas comosus* containing bromelain equivalent to gelatin digesting units (GDU) between 1 and 1000
5 mg of coenzyme Q10
50 mg dry extract from the root of *Curcuma longa* titered at 95% total curcuminoids
25 mg extract from flowers of *Tagetes erecta* titered at 20% lutein
0.5 mg of copper (from microencapsulated copper sulfate)
15 mg of resveratrol purified from roots of *Polygonum cuspidatum*
75 mg dry extract of bark of *Salix alba* titered at 25% salicin
10 mg dry extract from stigmas of *Crocus sativus* titered at 2% safranal
10 mg extract from flowers of *Tagetes erecta* titered at 5% zeaxanthin
6.25 mg of zinc (from zinc oxide).

### Composition C

20 mg of concentrated extract from *Haematococcus sp.* containing no less than 2.0% astaxanthin
2.5 mg dry extract, titered at 95% piperine obtained from fruits of *Piper nigrum*
100 mg of extract from gum resin of *Boswellia serrata* titered at
70% total boswellic acids and 20% beta boswellic acids
81 mg extract from stem and fruit of *Ananas comosus* containing bromelain equivalent to gelatin digesting units (GDU) between 1 and 1000
5 mg of coenzyme Q10
50 mg dry extract from the root of *Curcuma longa* titered at 95% total curcuminoids
25 mg extract from flowers of *Tagetes erecta* titered at 20% lutein
0.5 mg of copper (from microencapsulated copper sulfate)
15 mg of resveratrol purified from roots of *Polygonum cuspidatum*
75 mg dry extract of bark of *Salix alba* titered at 25% salicin
10 mg dry extract from stigmas of *Crocus sativus* titered at 2% safranal
10 mg extract from flowers of *Tagetes erecta* titered at 5% zeaxanthin
6.25 mg of zinc (from zinc oxide).
30 mg dry extract from *Ribes nigrum* with an oxygen radical absorption capacity (ORAC) of no less than 2 mmol trolox equivalents per gram of extract.

**Example 2:** retinal ischemia-reperfusion injury model in the rat. A model of retinal ischemia-reperfusion (IR) in rats was used, which is widely used in the prior art as a model of acute hypertension-induced retinal degeneration. Adult male Brown-Norway rats weighing about 200 g were pretreated orally once daily through tube feeding with composition C of example 1 (30 mg/day) or vehicle (HCV; Nutilis, 4.25%) . A pre-treatment was made for 5 days, a further treatment was made about an hour before induction of IR and the treatment was continued for the next 7 days. After five days of pretreatment, the animals were anesthetized intraperitoneally with a Zoletil (420 µ/Kg) and Atila (37 µl/Kg) solution and following mydriasis induced by instillation of 0.5% tropicamide, IR was induced by the insertion of a 30 gauge needle connected to a container of saline into the anterior chamber of the eye. The reserve of saline solution was placed at a height from the operating table such as to ensure hydrostatic pressure of about 130 mmHg at the needle. The intraocular pressure rise to levels which induce ischemia was confirmed by the "whitening" of the fundus. After 60 minutes, the needle was removed to allow the restoration of the retinal blood flow. In order to assess the damage induced by IR, analysis were conducted to highlight any variations in the retinal cell function by electroretinogram (ERG), using a Ganzfeld electroretinograph of Phoenix Research Laboratories. The electroretinogram was recorded in animals adapted to darkness for at least 12h and following anesthesia and mydriasis, two electrodes were inserted in cranial and caudal position, respectively, and the third at the Ganzfeld chamber. The ERG was recorded as the mean of ten green light flashes emitted for 1 millisecond (ms) at an intensity of 80 candles per second per square meter (cd·s/m²). The recording and analysis of the ERG conducted using the LabScribe software were performed before induction (Baseline), at 3 (T3) and 7 (T7) days after IR. In order to assess the retinal function, the amplitude of wave a (functionality of cone and rod photoreceptors) and wave b (Müller cell and bipolar cell functionality) were taken into account.

In the experiments conducted, a statistically significant reduction was observed in the amplitude of wave a (Figure 1A) and b (Figure 1B) in animals treated with VHC or Composition C at 3 days with respect to the Baseline, confirming the onset of the functional damage caused by the ischemic event. In contrast, 7 days after the induction of IR, the statistically significant reduction of the amplitude of wave a and wave b in VHC with respect to the Baseline is retained, but the significance is lost in animals treated with Composition C, indicating that the treatment is able to restore the retinal function to values comparable to the Baseline (Figure 1A and B, light circle).

A significant improvement of the retinal function of both waves a and b was also noted in the animals treated with Composition C with respect to those treated with vehicle at T7 after the induction of IR.

With the objective of evaluating a possible effect of the composition according to the present invention on the gene expression of TNFa, adult male Brown-Norway rats weighing about 200 g were treated once daily orally during the five days before and 1h before the induction of retinal IR with VHC (Nutilis, 4.25%) or with Composition C (30 mg/day). 6h after the ischemic event, the animals were sacrificed and the retinas taken and preserved in 50 µl of RNA later. The retina samples were subjected to RNA extraction using TRIzol. β-actin was used as endogenous control and the average ΔCt of naive controls (CTRL NAÏVE) was used as a calibrator. The results obtained by Real Time RT-PCR show that the treatment with Composition C can significantly reduce the retinal TNFa expression level with respect to HCV, thus showing a surprising natural anti-ischemic and antiedema effect (Figure 2) of the composition according to the present invention.

## Claims

1. A composition comprising: astaxanthin, piperine, boswellic acids, coenzyme Q10, curcuminoids, lutein, zeaxanthin, saffron, copper.

2. A composition according to claim 1, further comprising resveratrol, bromelain, zinc, salicin.

3. A composition according to one of claims 1 or 2, comprising further natural antioxidants, preferably polymeric anthocyanins.

4. A composition according to one of claims 1 to 3, comprising: 0.1 to 200 mg of concentrated extract from *Haematococcus sp.* containing no less than 2.0% astaxanthin, 0.01 to 15 mg dry extract, titered at 95% piperine obtained from fruits of *Piper nigrum,* 10 to 2000 mg of extract from gum resin of *Boswellia serrata* titered at 70% total boswellic acids and 20% beta boswellic acids, 0 to 500 mg of extract from stem and fruit of *Ananas comosus* containing bromelain equivalent to gelatin digesting units (GDU) of between 1 and 1000, 0.5 to 100 mg of coenzyme Q10, 1 to 500 mg dry extract from the root of *Curcuma longa* titered at 95% total curcuminoids, 0.1 to 250 mg extract from flowers of *Tagetes erecta* titered at 20% lutein, 0 to 500 mg dry extract from *Ribes nigrum* with an oxygen radical absorption capacity (ORAC) of no less than 2 mmol trolox equivalents per gram of extract, 0.25 to 0.75 mg of copper (from microencapsulated copper sulfate), 0 to 150 mg of resveratrol purified from roots of *Polygonum cuspidatum,* 0 to 200 mg dry extract of bark of *Salix alba* titered at 25% salicin, 1 to 100 mg of dry extract from stigmas of *Crocus sativus* titered at 2% safranal, 0.1 to 30 mg of extract from flowers of *Tagetes erecta* titered at 5% zeaxanthin, 0 to 10 mg of zinc (from zinc oxide).

5. A composition according to one of claims 1 to 4, comprising: 1 to 100 mg of concentrated extract from *Haematococcus sp.* containing no less than 2.0% astaxanthin, 0.2 to 8 mg dry extract, titered at 95% piperine obtained from fruits of *Piper nigrum,* 25 to 1000 mg of extract from gum resin of *Boswellia serrata* titered at 70% total boswellic acids and 20% beta boswellic acids, 5 to 300 mg of extract from stem and fruit of *Ananas comosus* containing bromelain equivalent to gelatin digesting units (GDU) of between 8 and 480, 3 to 75 mg of coenzyme Q10, 10 to 250 mg dry extract from the root of *Curcuma longa* titered at 95% total curcuminoids, 1 to 150 mg extract from flowers of *Tagetes erecta* titered at 20% lutein, 15 to 125 mg dry extract from *Ribes nigrum* with an oxygen radical absorption capacity (ORAC) of no less than 2 mmol trolox equivalents per gram of extract, 0.3 to 0.75 mg of copper (from microencapsulated copper sulfate), 3 to 75 mg of resveratrol purified from roots of *Polygonum cuspidatum, 30* to 100 mg dry extract of bark of *Salix alba* titered at 25% salicin, 4 to 40 mg of dry extract from stigmas of *Crocus sativus* titered at 2% safranal, 0.5 to 18 mg of extract from flowers of *Tagetes erecta* titered at 5% zeaxanthin, 3 to 7.5 mg of zinc (from zinc oxide).

6. A composition according to one of claims 1 to 5, comprising: 5 to 50 mg of concentrated extract from *Haematococcus sp.* containing no less than 2.0% astaxanthin, 0.75 to 4 mg dry extract, titered at 95% piperine obtained from fruits of *Piper nigrum,* 50 to 600 mg of extract from gum resin of *Boswellia serrata* titered at 70% total boswellic acids and 20% beta boswellic acids, 20 to 200 mg of extract from stem and fruit of *Ananas comosus* containing bromelain equivalent to gelatin digesting units (GDU) of between 15 and 370, 3 to 25 mg of coenzyme Q10, 30 to 100 mg dry extract from the root of *Curcuma longa* titered at 95% total curcuminoids, 10 to 50 mg extract from flowers of *Tagetes erecta* titered at 20% lutein, 20 to 60 mg dry extract from *Ribes nigrum* with an oxygen radical absorption capacity (ORAC) of no less than 2 mmol trolox equivalents per gram of extract, 0.35 to 0.65 mg of copper (from microencapsulated copper sulfate), 8 to 25 mg of resveratrol purified from roots of *Polygonum cuspidatum,* 35 to 95 mg dry extract of bark of *Salix alba* titered at 25% salicin, 6 to 20 mg of dry extract from stigmas of *Crocus sativus* titered at 2% safranal, 2 to 15 mg of extract from flowers of *Tagetes erecta* titered at 5% zeaxanthin, 4 to 7 mg of zinc (from zinc oxide).

7. A composition according to one of claims 1 to 6, comprising: 10 to 30 mg of concentrated extract from *Haematococcus sp.* containing no less than 2.0% astaxanthin, 1 to 3 mg dry extract, titered at 95% piperine obtained from fruits of *Piper nigrum,* 60 to 200 mg of extract from gum resin of *Boswellia serrata* titered at 70% total boswellic acids and 20% beta boswellic acids, 30 to 100 mg of extract from stem and fruit of *Ananas comosus* containing bromelain equivalent to gelatin digesting units (GDU) of between 50 and 200, 4 to 12 mg of coenzyme Q10, 30 to 70 mg dry extract from the root of *Curcuma longa* titered at 95% total curcuminoids, 15 to 35 mg extract from flowers of *Tagetes erecta* titered at 20% lutein, 20 to 40 mg dry extract from Ribes nigrum with an oxygen radical absorption capacity (ORAC) of no less than 2 mmol trolox equivalents per gram of extract, 0.4 to 0.6 mg of copper (from microencapsulated copper sulfate), 10 to 20 mg of resveratrol purified from roots of Polygonum cuspidatum, 40 to 90 mg dry extract of bark of Salix albatitered at 25% salicin, 7 to 15 mg of dry extract from stigmas of *Crocus sativus* titered at 2% safranal, 5 to 15 mg of extract from flowers of *Tagetes erecta* titered at 5° zeaxanthin, 5 to 7 mg of zinc (from zinc oxide).

8. A composition according to one of claims 1 to 7, for use in treating retinal diseases of an oedematous and/or ischemic and/or degenerative nature, preferably selected from the group consisting of: macular degeneration, diabetic retinopathy, retinal venous occlusions, central retinal vein occlusion, retinal branch vein occlusion, retinal arterial tree occlusion, central retinal arterial occlusion, branch retinal arterial occlusion, rhegmatogenous retinal degenerations, lattice or palisade degeneration, snail slime degeneration, degenerative retinoschisis, non-rhegmatogenous retinal degenerations, cystoid degeneration, snowflake degeneration, pavimentosa degeneration, honeycomb degeneration, peripheral drusen, oral pigmentary degeneration, hypertensive retinopathy, retinitis pigmentosa, maculopathy, Stargardt disease, central serous chorioretinopathy.

9. A composition for use according to claim 8 where said use is in the nutritional support to the treatment of macular degeneration, diabetic retinopathy and macular edema.

10. A composition for use according to claim 8 or 9, where said composition is administered to subjects exposed to treatment with anti-angiogenic agents.

11. A nutraceutical formulation comprising the composition according to one of claims 1 to 6 and pharmaceutically acceptable excipients for oral administration.

## Patentansprüche

1. Eine Zusammensetzung, aufweisend: Astaxanthin, Piperin, Boswelliasäuren, Coenzym Q10, Curcuminoide, Lutein, Zeaxanthin, Safran, Kupfer.

2. Eine Zusammensetzung nach Anspruch 1, ferner aufweisend Resveratrol, Bromelain, Zink, Salicin.

3. Eine Zusammensetzung nach einem der Ansprüche 1 oder 2, die weiteren natürlichen Antioxidantien, vorzugsweise polymere Anthocyane, aufweisend.

4. Eine Zusammensetzung nach einem der Ansprüche 1 bis 3, aufweisend: 0,1 bis 200 mg konzentriertes Extrakt aus Haematococcus sp., das nicht weniger als 2,0% Astaxanthin enthält, 0.01 bis 15 mg Trockenextrakt mit einem Titer von 95 % Piperin, erhalten aus Früchten von Piper nigrum, 10 bis 2000 mg Extrakt aus Gummiharz von Boswellia serrata mit einem Titer von 70 % Gesamtboswelliasäuren und 20 % Beta-Boswelliasäuren, 0 bis 500 mg Extrakt aus Stamm und Frucht der Ananas comosus, das Bromelain enthält, das Gelatineverdauungseinheiten (GDU) zwischen 1 und 1000 entspricht, 0.5 bis 100 mg Coenzym Q10, 1 bis 500 mg Trockenextrakt aus der Wurzel von Curcuma longa mit einem Titer von 95 % Gesamtcurcuminoiden, 0.1 bis 250 mg Extrakt aus Blüten von Tagetes erecta mit einem Titer von 20% Lutein, 0 bis 500 mg Trockenextrakt aus Ribes nigrum mit einer Sauerstoffradikalabsorptionskapazität (ORAC) von mindestens 2 mmol Troloxäquivalenten pro Gramm Extrakt, 0.25 bis 0.75 mg Kupfer (aus mikroverkapseltem Kupfersulfat), 0 bis 150 mg Resveratrol, gereinigt aus Wurzeln von Polygonum cuspidatum, 0 bis 200 mg Trockenextrakt aus Rinde von Salix alba mit einem Titer von 25% Salicin, 1 bis 100 mg Trockenextrakt aus Narben von Crocus sativus mit einem Titer von 2% Safranal, 0.1 bis 30 mg Extrakt aus Blüten von Tagetes erecta mit einem Titer von 5% Zeaxanthin, 0 bis 10 mg Zink (aus Zinkoxid).

5. Eine Zusammensetzung nach einem der Ansprüche 1 bis 4, aufweisend: 1 bis 100 mg konzentriertes Extrakt aus Haematococcus sp., das nicht weniger als 2,0% Astaxanthin enthält, 0.2 bis 8 mg Trockenextrakt mit einem Titer von 95 % Piperin, erhalten aus Früchten von Piper nigrum, 25 bis 1000 mg Extrakt aus Gummiharz von Boswellia serrata mit einem Titer von 70 % Gesamtboswelliasäuren und 20 % Beta-Boswelliasäuren, 5 bis 300 mg Extrakt aus Stamm und Frucht der Ananas comosus, das Bromelain, enthält das Gelatineverdauungseinheiten (GDU) zwischen 8 und 480 entspricht, 3 bis 75 mg Coenzym Q10, 10 bis 250 mg Trockenextrakt aus der Wurzel von Curcuma longa mit einem Titer von 95 % Gesamtcurcuminoiden, 1 bis 150 mg Extrakt aus Blüten von Tagetes erecta mit einem Titer von 20 % Lutein, 15 bis 125 mg Trockenextrakt aus Ribes nigrum mit einer Sauerstoffradikalabsorptionskapazität (ORAC) von mindestens 2 mmol Troloxäquivalenten pro Gramm Extrakt, 0.3 bis 0.75 mg Kupfer (aus mikroverkapseltem Kupfersulfat), 3 bis 75 mg Resveratrol, gereinigt aus Wurzeln von Polygonum cuspidatum, 30 bis 100 mg Trockenextrakt aus Rinde von Salix alba, mit einem Titer von 25% Salicin , 4 bis 40 mg Trockenextrakt aus Narben von Crocus sativus, mit einem Titer von 2% Safranal ist, 0.5 bis 18 mg Extrakt aus Blüten von Tagetes erecta, mit einem Titer von 5 % Zeaxanthin, 3 bis 7.5 mg Zink (aus Zinkoxid).

6. Eine Zusammensetzung nach einem der Ansprüche 1 bis 5, aufweisend: 5 bis 50 mg konzentriertes Extrakt aus Haematococcus sp., das nicht weniger als 2,0% Astaxanthin enthält, 0.75 bis 4 mg Trockenextrakt mit einem Titer von 95 % Piperin, erhalten aus Früchten von Piper nigrum, 50 bis 600 mg Extrakt aus Gummiharz von Boswellia serrata mit einem Titer von 70 % Gesamtboswelliasäuren und 20 % Beta-Boswelliasäuren, 20 bis 200 mg Extrakt aus Stamm und Frucht der Ananas comosus, das Bromelain enthält, das Gelatineverdauungseinheiten (GDU) zwischen 15 und 370 entspricht, 3 bis 25 mg Coenzym Q10, 30 bis 100 mg Trockenextrakt aus der Wurzel von Curcuma longa mit einem Titer von 95 % Gesamtcurcuminoiden, 10 bis 50 mg Extrakt aus Blüten von Tagetes erecta mit einem Titer von 20 % Lutein, 20 bis 60 mg Trockenextrakt aus Ribes nigrum mit einer Sauerstoffradikalabsorptionskapazität (ORAC) von mindestens 2 mmol Troloxäquivalenten pro Gramm Extrakt, 0.35 bis 0.65 mg Kupfer (aus mikroverkapseltem Kupfersulfat), 8 bis 25 mg Resveratrol, gereinigt aus Wurzeln von Polygonum cuspidatum, 35 bis 95 mg Trockenextrakt aus Rinde von Salix alba mit einem Titer von 25 % Salicin, 6 bis 20 mg Trockenextrakt aus Narben von Crocus sativus mit einem Titer von 2 % Safranal, 2 bis 15 mg Extrakt aus Blüten von Tagetes erecta mit einem Titer von 5 % Zeaxanthin, 4 bis 7 mg Zink (aus Zinkoxid).

7. Eine Zusammensetzung nach einem der Ansprüche 1 bis 6, aufweisend: 10 bis 30 mg konzentriertes Extrakt aus Haematococcus sp, das nicht weniger als 2.0 % Astaxanthin enthält, 1 bis 3 mg Trockenextrakt mit einem Titer von 95 % Piperin, erhalten aus Früchten von Piper nigrum, 60 bis 200 mg Extrakt aus Gummiharz von Boswellia serrata mit einem Titer von 70 % Gesamtboswelliasäuren und 20 % Beta-Boswelliasäuren, 30 bis 100 mg Extrakt aus Stamm und Frucht von Ananas comosus, das Bromelain, enthält, das Gelatineverdauungseinheiten (GDU) zwischen 50 und 200 entspricht, 4 bis 12 mg Coenzym Q10, 30 bis 70 mg Trockenextrakt aus der Wurzel von Curcuma longa mit einem Titer von 95 % Gesamtcurcuminoiden, 15 bis 35 mg Extrakt aus Blüten von Tagetes erecta mit einem Titer von 20 % Lutein, 20 bis 40 mg Trockenextrakt aus Ribes nigrum mit einer Sauerstoffradikalabsorptionskapazität (ORAC) von mindestens 2 mmol Troloxäquivalenten pro Gramm Extrakt, 0.4 bis 0.6 mg Kupfer (aus mikroverkapseltem Kupfersulfat), 10 bis 20 mg Resveratrol, gereinigt aus Wurzeln von Polygonum cuspidatum, 40 bis 90 mg Trockenextrakt aus Rinde von Salix alba mit einem Titer von 25% Salicin, 7 bis 15 mg Trockenextrakt aus Narben von Crocus sativus mit einem Titer von 2% Safranal, 5 bis 15 mg Extrakt aus Blüten von Tagetes erecta mit einem Titer von 5% Zeaxanthin, 5 bis 7 mg Zink (aus Zinkoxid).

8. Eine Zusammensetzung nach einem der Ansprüche 1 bis 7 zur Verwendung bei der Behandlung von Netzhauterkrankungen ödematöser und/oder ischämischer und/oder degenerativer Natur, vorzugsweise ausgewählt aus der Gruppe bestehend aus: Makuladegeneration, diabetische Retinopathie, retinale Venenverschlüsse, zentrale retinale Venenverschlüsse, retinale Venenastverschlüsse, retinale Arterienbaumverschlüsse, zentrale retinale Arterienverschlüsse, retinale Arterienastverschlüsse, rhegmatogene Netzhautdegenerationen, Gitter- oder Palisadendegenerationen, Schneckenschleimdegenerationen, degenerative Retinoschisis, nicht-rhegmatogene Netzhautdegenerationen, zystoide Degeneration, Schneeflockendegeneration, Pavimentosa-Degeneration, wabenförmige Degeneration, periphere Drusen, orale Pigmentdegeneration, hypertensive Retinopathie, Retinitis pigmentosa, Makulopathie, Stargardt-Krankheit, zentrale seröse Chorioretinopathie.

9. Eine Zusammensetzung zur Verwendung nach Anspruch 8, wobei die Verwendung in der Ernährungsunterstützung der Behandlung von Makuladegeneration, diabetischer Retinopathie und Makulaödem besteht.

10. Eine Zusammensetzung zur Verwendung nach Anspruch 8 oder 9, wobei die Zusammensetzung an Personen verabreicht wird, die einer Behandlung mit antiangiogenen Mitteln ausgesetzt sind.

11. Eine Nutrazeutische Formulierung, umfassend die Zusammensetzung nach einem der Ansprüche 1 bis 6 und pharmazeutisch annehmbare Hilfsstoffe zur oralen Verabreichung.

## Revendications

1. Composition comprenant : astaxanthine, pipérine, acides boswelliques, coenzyme Q10, curcuminoïdes, lutéine, zéaxanthine, safran, cuivre.

2. Composition selon la revendication 1, comprenant en outre du resvératrol, de la bromélaïne, du zinc, de la salicine.

3. Composition selon l'une des revendications 1 ou 2, comprenant en outre d'autres antioxydants naturels, de préférence des anthocyanes polymères.

4. Composition selon l'une des revendications 1 à 3, comprenant : 0,1 à 200 mg d'extrait concentré de *Haematococcus sp.* contenant au moins 2,0 % d'astaxanthine, 0,01 à 15 mg d'extrait sec, titré à 95 % de pipérine obtenue à partir des fruits de *Piper nigrum,* 10 à 2000 mg d'extrait de gomme-résine de *Boswellia serrata* titré à 70 % d'acides boswelliques totaux et à 20 % d'acides bêta boswelliques, 0 à 500 mg d'extrait de tige et de fruit *d'Ananas comosus* contenant de la bromélaïne équivalant à des unités de digestion de la gélatine (GDU) comprises entre 1 et 1000, 0,5 à 100 mg de coenzyme Q10, 1 à 500 mg d'extrait sec de racine de *Curcuma longa* titré à 95 % de curcuminoïdes totaux, 0,1 à 250 mg d'extrait de fleurs de *Tagetes erecta* titré à 20 % de lutéine, 0 à 500 mg d'extrait sec de *Ribes nigrum* avec une capacité d'absorption des radicaux oxygénés (ORAC) d'au moins 2 mmol d'équivalents trolox par gramme d'extrait, 0,25 à 0,75 mg de cuivre (provenant de sulfate de cuivre microencapsulé), 0 à 150 mg de resvératrol purifié à partir de racines de *Polygonum cuspidatum,* 0 à 200 mg d'extrait sec d'écorce de *Salix alba* titré à 25 % de salicine, 1 à 100 mg d'extrait sec de stigmates de *Crocus sativus* titré à 2 % de safranal, 0,1 à 30 mg d'extrait de fleurs de *Tagetes erecta* titré à 5 % de zéaxanthine, 0 à 10 mg de zinc (provenant de l'oxyde de zinc).

5. Composition selon l'une des revendications 1 à 4, comprenant : 1 à 100 mg d'extrait concentré de *Haematococcus sp.* contenant au moins 2,0 % d'astaxanthine, 0,2 à 8 mg d'extrait sec, titré à 95 % de pipérine obtenue à partir des fruits de *Piper nigrum,* 25 à 1000 mg d'extrait de gomme-résine de *Boswellia serrata* titré à 70 % d'acides boswelliques totaux et 20 % d'acides bêta boswelliques, 5 à 300 mg d'extrait de tige et de fruit *d'Ananas comosus* contenant de la bromélaïne équivalant à des unités de digestion de la gélatine (GDU) comprises entre 8 et 480, 3 à 75 mg de coenzyme Q10, 10 à 250 mg d'extrait sec de racine de *Curcuma longa* titré à 95 % de curcuminoïdes totaux, 1 à 150 mg d'extrait de fleurs de *Tagetes erecta* titré à 20 % de lutéine, 15 à 125 mg d'extrait sec de *Ribes nigrum* avec une capacité d'absorption des radicaux oxygénés (ORAC) d'au moins 2 mmol d'équivalents trolox par gramme d'extrait, 0,3 à 0,75 mg de cuivre (provenant de sulfate de cuivre microencapsulé), 3 à 75 mg de resvératrol purifié à partir de racines de *Polygonum cuspidatum,* 30 à 100 mg d'extrait sec d'écorce de *Salix alba* titré à 25 % de salicine, 4 à 40 mg d'extrait sec de stigmates de *Crocus sativus* titré à 2 % de safranal, 0,5 à 18 mg d'extrait de fleurs de *Tagetes erecta* titré à 5 % de zéaxanthine, 3 à 7,5 mg de zinc (provenant de l'oxyde de zinc).

6. Composition selon l'une des revendications 1 à 5, comprenant : 5 à 50 mg d'extrait concentré de *Haematococcus sp.* contenant au moins 2,0 % d'astaxanthine, 0,75 à 4 mg d'extrait sec, titré à 95 % de pipérine obtenue à partir des fruits de *Piper nigrum,* 50 à 600 mg d'extrait de gomme-résine de *Boswellia serrata* titré à 70 % d'acides boswelliques totaux et 20 % d'acides bêta boswelliques, 20 à 200 mg d'extrait de tige et de fruit *d'Ananas comosus* contenant de la bromélaïne équivalant à des unités de digestion de la gélatine (GDU) comprises entre 15 et 370, 3 à 25 mg de coenzyme Q10, 30 à 100 mg d'extrait sec de racine de *Curcuma longa* titré à 95 % de curcuminoïdes totaux, 10 à 50 mg d'extrait de fleurs de *Tagetes erecta* titré à 20 % de lutéine, 20 à 60 mg d'extrait sec de *Ribes nigrum* avec une capacité d'absorption des radicaux oxygénés (ORAC) d'au moins 2 mmol d'équivalents trolox par gramme d'extrait, 0,35 à 0,65 mg de cuivre (provenant de sulfate de cuivre microencapsulé), 8 à 25 mg de resvératrol purifié à partir de racines de *Polygonum cuspidatum,* 35 à 95 mg d'extrait sec d'écorce de *Salix alba* titré à 25 % de salicine, 6 à 20 mg d'extrait sec de stigmates de *Crocus sativus* titré à 2 % de safranal, 2 à 15 mg d'extrait de fleurs de *Tagetes erecta* titré à 5 % de zéaxanthine, 4 à 7 mg de zinc (provenant de l'oxyde de zinc).

7. Composition selon l'une des revendications 1 à 6, comprenant : 10 à 30 mg d'extrait concentré de *Haematococcus sp.* contenant au moins 2.0 % d'astaxanthine, 1 à 3 mg d'extrait sec titré à 95 % de pipérine obtenue à partir des fruits de *Piper nigrum,* 60 à 200 mg d'extrait de gomme-résine de *Boswellia serrata* titré à 70 % d'acides boswelliques totaux et 20 % d'acides bêta boswelliques, 30 à 100 mg d'extrait de tige et de fruit *d'Ananas comosus* contenant de la bromélaïne équivalant à des unités de digestion de la gélatine (GDU) comprises entre 50 et 200, 4 à 12 mg de coenzyme Q10, 30 à 70 mg d'extrait sec de racine de *Curcuma longa* titré à 95 % de curcuminoïdes totaux, 15 à 35 mg d'extrait de fleurs de *Tagetes erecta* titré à 20 % de lutéine, 20 à 40 mg d'extrait sec de Ribes nigrum avec une capacité d'absorption des radicaux oxygénés (ORAC) d'au moins 2 mmol d'équivalents trolox par gramme d'extrait, 0,4 à 0,6 mg de cuivre (provenant de sulfate de cuivre microencapsulé), 10 à 20 mg de resvératrol purifié à partir de racines de Polygonum cuspidatum, 40 à 90 mg d'extrait sec d'écorce de Salix alba titré à 25 % de salicine, 7 à 15 mg d'extrait sec de stigmates de *Crocus sativus* titré à 2 % de safranal, 5 à 15 mg d'extrait de fleurs de *Tagetes erecta* titré à 5 % de zéaxanthine, 5 à 7 mg de zinc (provenant de l'oxyde de zinc).

8. Composition selon l'une des revendications 1 à 7, pour une utilisation dans le traitement des maladies rétiniennes de nature oedémateuse et/ou ischémique et/ou dégénérative, de préférence choisies dans le groupe consistant de : dégénérescence maculaire, rétinopathie diabétique, occlusions veineuses rétiniennes, occlusion de la veine centrale de la rétine, occlusion de la veine de branche de la rétine, occlusion de l'arbre artériel rétinien, occlusion artérielle centrale de la rétine, occlusion artérielle de branche de la rétine, dégénérescences rétiniennes rhegmatogènes, dégénérescence en treillis ou palissadique, dégénérescence en bave d'escargot, rétinoschisis dégénératif, dégénérescences rétiniennes non rhégmatogènes, dégénérescence cystoïde, dégénérescence en flocon de neige, dégénérescence en pavimentosa, dégénérescence en nid d'abeille, drusen périphériques, dégénérescence pigmentaire orale, rétinopathie hypertensive, rétinite pigmentaire, maculopathie, maladie de Stargardt, choriorétinopathie séreuse centrale.

9. Composition pour une utilisation selon la revendication 8 où ladite utilisation est le soutien nutritionnel au traitement de la dégénérescence maculaire, de la rétinopathie diabétique et de l'œdème maculaire.

10. Composition pour une utilisation selon la revendication 8 ou 9, où ladite composition est administrée à des sujets exposés à un traitement avec des agents anti-angiogéniques.

11. Formulation nutraceutique comprenant la composition selon l'une des revendications 1 à 6 et des excipients pharmaceutiquement acceptables pour l'administration orale.
